# EUROPÄISCHE PATENTSCHRIFT

(11) **EP 0 724 870 B1**
(45) Veröffentlichungstag und Bekanntmachung des Hinweises auf die Patenterteilung: **27.12.2000**
(21) Anmeldenummer: 96101227.5
(22) Anmeldetag: 29.01.1996
(51) Int. Cl.: A61F 13/15

(54) **Wegwerfwindel**
Disposable diaper
Couche jetable

(30) Priorität: 31.01.1995 JP 1383695
(43) Veröffentlichungstag der Anmeldung: 07.08.1996
(73) Patentinhaber: UNI-CHARM CORPORATION, Kawanoe-shi Ehime-ken (JP)
(72) Erfinder: Inoue, Yasushi, Kannonji-shi, Kagawa-ken (JP); Kido, Tsutomo, Kawanoe-shi, Ehime-ken (JP)
(74) Vertreter: Sperling, Rüdiger, Dipl.-Ing.

(56) Entgegenhaltungen:
- EP-A- 0 343 941
- WO-A-86/05089
- WO-A-94/06385
- GB-A- 2 055 297
- GB-A- 2 252 047
- GB-A- 2 255 720

## Beschreibung

Die vorliegende Erfindung betrifft eine Wegwerfwindel, die zum Aufsaugen und Aufnehmen von Urin und anderen körperlichen Ausscheidungen verwendet wird.

Bei Wegwerfwindeln ist es wohlbekannt, einen flüssigkeitsabsorbierenden Kern vorzusehen, der eine Füllung, die eine bauschige oder Flaumpulpe und superabsorbierende Polymerteilchen enthält, und eine faserige Gewebelage umfaßt, die die Füllung bedeckt, um einen Dämpfungseffekt zu erreichen und einmal absorbierte körperliche Ausscheidungen am Zurückfließen zu hindern. Zum Beispiel offenbart die japanische offengelegte Gebrauchsmusteranmeldung Nr. Sho52-99046 eine wegwerfbare absorbierende Wattierung, gemäß der eine flüssigkeitshaltende Lage aus Pulpenfasern und superabsorbierenden Polymerteilchen mit 10 bis 60 Gewichtsprozent besteht, dann eine Lage aus Seiden- oder gewebeartigem Papier und eine kurzfaserige Flusen- oder Flaumlage nacheinander auf eine obere Oberfläche der flüssigkeitshaltenden Lage laminiert sind, und erforderlichenfalls das Laminat insgesamt mit einem Oberflächenmaterial, wie Textilverbundstoffen, bedeckt ist, um einen flüssigkeitsabsorbierenden Kern zu bilden. Die Flaum- oder Flusenlage entspricht der faserigen Gewebelage. Zusätzlich offenbart die japanische Patentveröffentlichung Nr. Hei6-38818 eine Wegwerfwindel, gemäß der eine bauschigen oder Flaumpulpeneinheit, die superabsorbierende Polymerteilchen enthält, mit einem Seiden- oder Tissue-Papier bedeckt ist, um eine flüssigkeitshaltende Lage zu bilden, und eine faserige Gewebelage, die einen gewünschten Druckelastizitäts-Regenerierungs- oder Erholungsfaktor hat, ist auf eine obere Oberfläche der flüssigkeitshaltenden Lage laminiert.

Jedoch weist im Fall der oben angegebenen Wattierung oder Windel die flüssigkeitshaltende Lage, die die superabsorbierenden Polymerteilchen mit 30 oder mehr Gewichtsprozent und die Pulpenfasern mit weniger als 70 Gewichtsprozent enthält, einen Nachteil darin auf, daß sich die Polymerteilchen unter eine adäquate Verflechtung unter den Pulpenfasern mischen und eine Flexibilität sowie eine Formstabilität verschlechtern. Wenn der flüssigkeitsabsorbierende Kern, der eine derartige flüssigkeitshaltende Lage enthält, verwendet wird, wird die flüssigkeitshaltende Lage leicht lose oder lockert sich, wenn die Windel auf Grund aktiver Bewegungen des Babys, das die Wattierung oder die Windel trägt, verformt wird, und folglich können sich dichte Kontakte der flüssigkeitshaltenden Lage, der faserigen Gewebelage und des Tissue- oder gewebeartigen Papiers lösen, und dies erschwert eine schnelle Absorption von körperlichen Ausscheidungen. Innerhalb der flüssigkeitshaltenden Lage werden enge Kontakte von Zwischenfasern sowie die von den Fasern und den Polymerteilchen verschlechtert, und eine stetige Diffusion von körperlichen Ausscheidungen wird verhindert.

Entsprechend ist es ein grundsätzliches Ziel der Erfindung, die oben angegebenen Probleme zu lösen.

Dieses Ziel wird durch eine Wegwerfwindel nach dem Anspruch 1 erreicht. Vorteilhafte und bevorzugte Weiterbildungen der Erfindung ergeben sich aus den Unteransprüchen im einzelnen und deren Kombinationen.

Das vorstehend angegebene Ziel wird gemäß der Erfindung mit einer Wegwerfwindel erreicht, die eine flüssigkeitsdurchlässige Decklage, eine flüssigkeitsundurchlässige Außenlage und einen zwischen diesen beiden Lagen angeordneten flüssigkeitsabsorbierenden Kern enthält, der aus einer flüssigkeitshaltenden Lage, die bauschige oder Flaumpulpe mit weniger als 70 Gewichtsprozent und superabsorbierende Polymerteilchen mit 30 oder mehr Gewichtsprozent enthält, und einer isolierenden Faserlage besteht, die die flüssigkeitshaltende Lage bedeckt, wobei der flüssigkeitsabsorbierende Kern die flüssigkeitshaltenden Lage und die isolierende Faserlage enthält, die vollständig mit einer flüssigkeitsdurchlässigen Deckschicht bedeckt sind, und die flüssigkeitshaltende Lage, die isolierende Faserlage und die flüssigkeitsdurchlässige Deckschicht im wesentlichen über die gesamten Ausdehnungen der jeweiligen Laminierungsoberflächen intermittierend miteinander verbunden sind.

Gemäß einer Ausführung der Erfindung enthält die isolierende Faserlage einen durch einen Schmelzvorgang verbundenen Textilverbundstoff oder Gewebeverbund, und ein Heißkleber oder thermoplastischer Klebstoff wird als das Bindemittel verwendet. Vorzugsweise hat die isolierende Faserlage eine Dichte, die niedriger als jene der bauschigen oder Flusenpulpe ist, die in der flüssigkeitshaltenden Lage enthalten ist. Die isolierende Faserlage kann aus einer Mischung aus hydrophilen Fasern und hydrophoben Fasern gebildet sein. Die flüssigkeitsdurchlässige Deckschicht kann hydrophil sein und ein Körperausscheidungs-Diffusionsvermögen bieten, das höher als jenes ist, das von der isolierenden Faserlage geboten wird.

Bei der oben beschriebenen Wegwerfwindel besteht kein Besorgnis, daß der flüssigkeitsabsorbierende Kern leicht aus der Form geraten könnte, und die flüssigkeitshaltende Lage, die isolierende Faserlage und die flüssigkeitsdurchlässige Deckschicht können zuverlässig miteinander verbunden bleiben, selbst wenn die flüssigkeitshaltende Lage eine relativ große Menge von Polymerteilchen enthält, da die flüssigkeitshaltende Lage, die isolierende Faserlage und die flüssigkeitsdurchlässige Deckschicht im wesentlichen über die gesamten Ausdehnungen der jeweiligen Laminierungsoberflächen intermittierend miteinander verbunden sind. Körperausscheidungen, die auf die Decklage abgegeben werden, durchdringen diese, diffundieren dann in die Deckschicht und durchdringen schnell die isolierende Faserlage in die flüssigkeitshaltende Lage hinein, von der Körperausscheidungen absorbiert und zurückgehalten werden. Die isolierende Faserlage kann dazu dienen, die flüssigkeitshaltende Lage, die dadurch absorbierte Körperausscheidungen enthält, von der Haut des Trägers fern zu halten.

Die Erfindung wird nachfolgend anhand von Ausführungsbeispielen unter Bezugnahme auf die zugehörigen Zeichnungen näher beschrieben, in denen:
Fig. 1 eine perspektivische Ansicht ist, die eine Wegwerfwindel in teilweise aufgebrochener Ansicht zeigt;
Fig. 2 eine Schnittansicht längs einer Linie II-II in der Fig. 1 ist; und
Fig. 3 eine Draufsicht einer flüssigkeitshaltenden Lage ist, die exemplarisch verschiedene Kleber-Beschichtungsmuster (A) bis (E) zeigt.

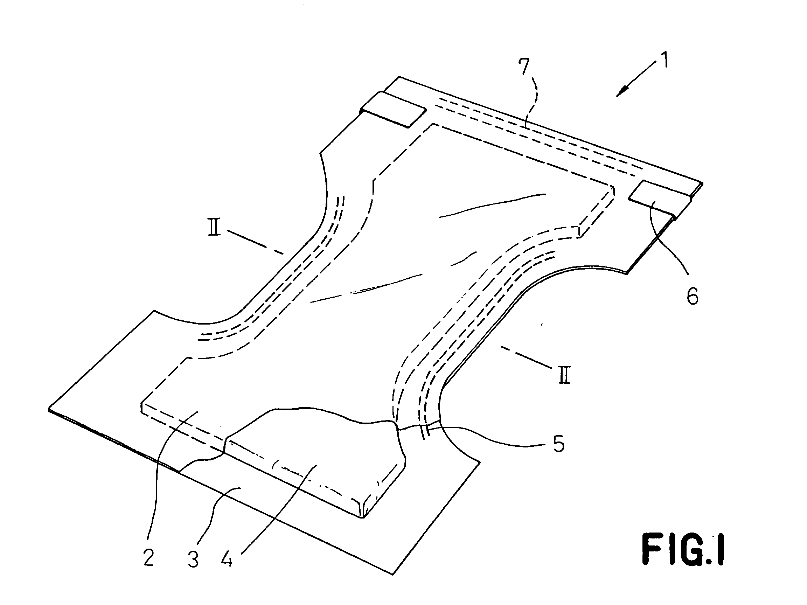

Unter Bezugnahme auf die Fig. 1 enthält eine Windel 1 eine flüssigkeitsdurchlässige Decklage 2, eine flüssigkeitsundurchlässige Außenlage 3, einen flüssigkeitsabsorbierenden Kern 4, der zwischen diesen beiden Lagen 2, 3 angeordnet ist, und für Beinöffnungen Elastikelemente 5, die unter Spannung mit einer Innenfläche oder mit Innenflächen der Decklage 2 und/oder der Außenlage 3 längs quer entgegengesetzten gekrümmten Seitenränder der Windel 1 verbunden sind. Ein Hinterteil der Windel 1 ist an seinen quer entgegengesetzten Seiten mit herkömmlichen Befestigungsbändern 6 versehen, die daran angebracht sind, und die Windel 1 ist ferner längs in Längsrichtung entgegengesetzten Enden für eine Hüftöffnung mit elastischen Elementen 7 versehen, die unter Spannung mit einer Innenfläche oder mit Innenflächen der Decklage 2 und/oder der Außenlage 3 verbunden sind.

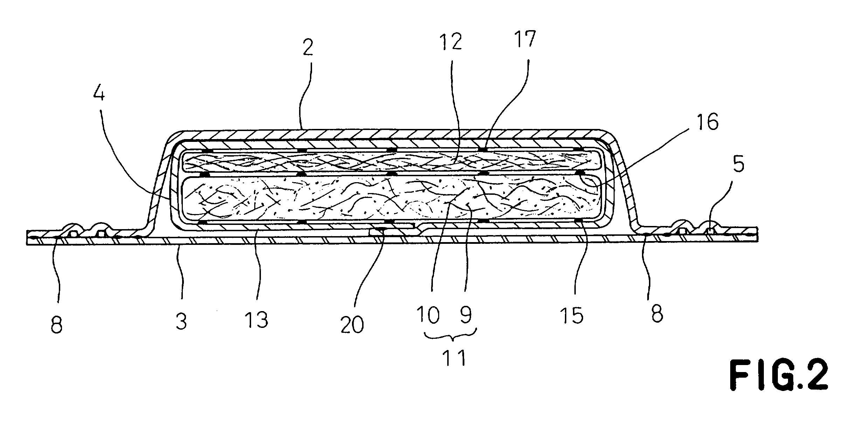

Unter Bezugnahme auf die Fig. 2 sind die Deck- und Außenlagen 2, 3 längs ihren Abschnitten, die auswärts über einen Umfangsrand des Kerns 4 hinaus verlaufen, aufeinander angeordnet und mittels eines Heißklebers 8 miteinander verbunden. Der Kern 4 enthält eine flüssigkeitshaltende Lage 11, die durch Preßformen einer Mischung aus super- oder hochabsorbierenden Polymerteilchen 9 mit 30 oder mehr Gewichtsprozent und einer Flaum- oder Flusenpulpe 10 mit weniger als 70 Gewichtsprozent erhalten wird, eine isolierende Faserlage 12, die aus einem durch einen Schmelzvorgang verbundenen Textilverbundstoff oder Verbundgewebe besteht und in Form und Größe identisch mit der flüssigkeitshaltenden Lage 11 und darauf angeordnet ist, und eine flüssigkeitsdurchlässige Deckschicht 13, wie ein Tissue- oder gewebeartiges Papier, die diese beiden Schichten 11 und 12 vollständig bedeckt. Diese flüssigkeitshaltende Lage 11, isolierende Faserlage 12 und flüssigkeitsdurchlässige Deckschicht 13 sind zusammenlaminiert und durch einen Heißkleber 15, 16, 17 miteinander verbunden, der intermittierend und gleichmäßig auf die jeweiligen Laminierungsoberflächen aufgebracht ist.

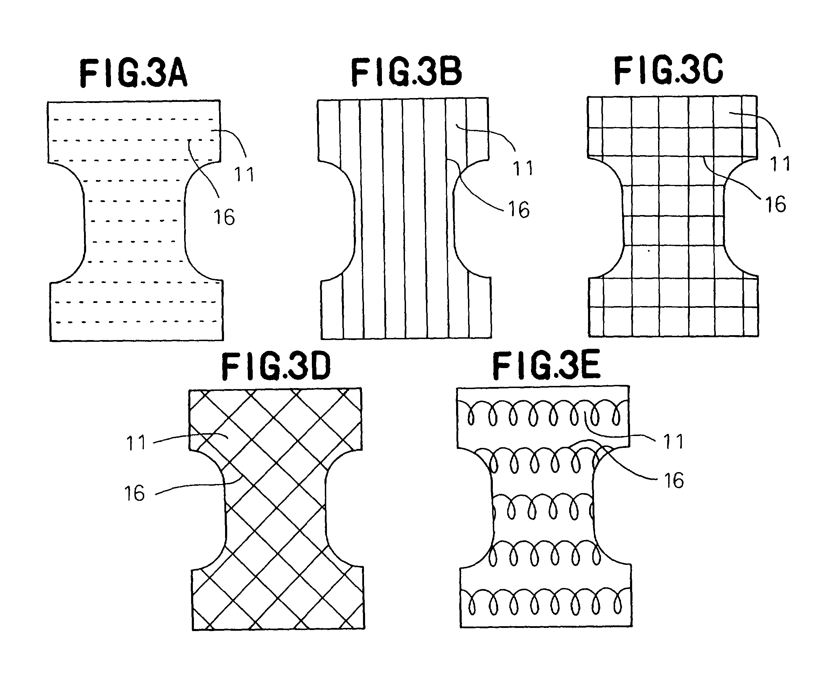

Wie in der Fig. 3 gezeigt ist, kann der Kleber 15, 16, 17 verschieden gemustert sein. Die flüssigkeitshaltende Lage 11 ist sanduhrförmig und der Kleber 15, 16, 17 kann in gepunkteten (A), vertikal gestreiften (B), gekreuzten (C) und (D) oder Spiralmustern (E) aufgebracht sein.

Wenn von dem Kleber 15, 16, 17 eine Mehrzahl von ununterbrochenen Linien beschrieben wird, muß jedes Paar benachbarter Linien ausreichend voneinander entfernt sein, um sicherzustellen, daß die Diffusion und Permeation durch sie nicht gestört wird. Relativ zu einer Fläche der flüssigkeitshaltenden Lage 11 wird der Kleber 15, 16, 17 jeweils vorzugsweise über 2 bis 30 % und noch bevorzugter über 5 bis 20 % davon aufgebracht.

Bei der Windel 1 der Erfindung können die Deck- und Außenlagen 2, 3 aus Materialien bestehen, die in dem relevanten technischen Gebiet üblicherweise verwendet werden. Die flüssigkeitshaltende Lage 11 kann, nicht nur um eine Körperausscheidungs-Diffsionsfähigkeit innerhalb der Schicht zu verbessern, sondern auch, um es zu gestatten, daß die flüssigkeitshaltende Lage 11 thermisch geformt wird, mit thermoplastischen Synthetikfasern bis zu 20 Gewichtsprozent gemischt sein. Die isolierende Faserlage 12 kann zum Isolieren der Decklage 4 von der flüssigkeitshaltenden Lage 11 dienen, und kann dadurch einen Rückfluß und eine Abgabe von Körperausscheidungen, die einmal von der flüssigkeitshaltenden Lage 11 absorbiert wurden, in die Decklage 2 unterdrücken, wenn der Kern 4 gegen den Träger gedrückt wird, und versieht den Kern 4 mit einer erwünschten Dämpfungsnatur, um eine harte Berührung der harten Polymerteilchen 9 zu lindern und gleichzeitig die Teilchen 9 daran zu hindern, durch die Deckschicht 13 hindurchzugehen. Zusätzlich erleichtert es die isolierende Faserlage 12 Körperausscheidungen, in der Horizontalrichtung des Kerns 4 zu diffundieren. Die isolierende Faserlage 12 kann aus hydrophilen Synthetikfasern oder einer Mischung aus solchen Fasern und hydrophoben thermoplatischen Synthetikfasern bestehen. Obwohl die isolierende Faserlage 12 vorzugsweise aus einem durch einen Schmelzvorgang verbundenen Textilverbundstoff oder Verbundgewebe gebildet ist, ist es auch möglich, den Textilverbundstoff durch ein Fasergewebe zu ersetzen, das nur einem Kardieren unterzogen wurde. Die Deckschicht 13 enthält als seinen Hauptbestandteil ein hydrophiles Material, wie Holzstoff oder Viskosefilamentfasern. Die Deckschicht 13 ist vorzugsweise ausgelegt, eine Körperausscheidungs-Diffusionsfähigkeit zu bieten, die höher als jene ist, die von der Fasergewebelage 12 geboten wird, und die gesamte Oberfläche des Kerns 4 zu bedecken, so daß die Polymerteilchen 9 zuverlässig darin eingeschlossen sind. Zu diesem Zweck können quer entgegengesetzte Seitenränder der Deckschicht 13 aufeinandergelegt und an der Unterseite der flüssigkeitshaltenden Lage 11 mittels Heißkleber verbunden werden, oder, obwohl es nicht gezeigt ist, können in Längsrichtung entgegengesetzte Enden davon aufeinandergelegt und miteinander verbunden werden. Alternativ kann ein geeignetes Lagenmaterial zwischen der Unterseite der flüssigkeitshaltenden Lage 11 und der Deckschicht 13 angeordnet werden, um die Polymerteilchen 9 am Durchbrechen oder Hindurchgehen durch die Deckschicht 13 zu hindern, die unter der flüssigkeitshaltenden Lage 11 liegt. Eine Dichte der Fasergewebeschicht 12 kann so eingestellt werden, daß sie höher als jene der Pulpe 10 ist, um eine Abwärtsbewegung der körperlichen Ausscheidungen zu erleichtern.

Bei der Wegwerfwindel der Erfindung sind die flüssigkeitshaltende Lage, die isolierende Lage und die Deckschicht, die zusammenlaminiert sind, um den flüssigkeitsabsorbierenden Kern zu bilden, über die gesamten Ausdehnungen der jeweiligen Laminierungsoberflächen intermittierend miteinander verbunden, so daß diese laminierten Lagen in einem zuverlässig verbundenen Zustand gehalten werden können, selbst wenn die an den Körper des Trägers angelegte Windel gekrümmt oder verformt wird. Eine relativ große Menge von superabsorbierenden Polymerteilchen kann ohne eine Besorgnis verwendet werden, daß die flüssigkeitshaltende Lage weniger flexibel wird und sich leicht ablöst oder lockert, was zu einem Ausder-Form-Geraten oder Formverlust führt. Auf diese Weise kann die erfindungsgemäße Windel eine erwünschte Absorptionsfähigkeit und einen Tragekomfort beibehalten, selbst wenn eine relativ große Menge von superabsorbierenden Polymerteilchen verwendet wird.

Zusammenfassend wird durch die Erfindung eine Wegwerfwindel geschaffen, die einen flüssigkeitsabsorbierenden Kern hat, der wiederum bauschige Pulpe mit weniger als 70 Gewichtsprozent und superabsorbierende Polymerteilchen mit 30 oder mehr Gewichtsprozent enthält, und bei der eine isolierende Faserlage auf einer oberen Oberfläche der flüssigkeitshaltenden Lage angeordnet ist. Dann sind diese flüssigkeitshaltende Lage und isolierende Faserlage vollständig mit einer flüssigkeitsdurchlässigen Deckschicht, wie einem Tissue-Papier, bedeckt, und diese Lagen sind über die gesamten Ausdehnungen der jeweiligen Laminierungsoberflächen intermittierend miteinander verbunden.

## Patentansprüche

1. Wegwerfwindel, enthaltend eine flüssigkeitsdurchlässige Decklage, eine flüssigkeitsundurchlässige Außenlage und einen zwischen diesen beiden Lagen angeordneten flüssigkeitsabsorbierenden Kern, der aus einer flüssigkeitshaltenden Lage, die bauschige Pulpe mit weniger als 70 Gewichtsprozent und superabsorbierende Polymerteilchen mit 30 oder mehr Gewichtsprozent enthält, und einer isolierenden Faserlage besteht, die die flüssigkeitshaltende Lage bedeckt,
dadurch gekennzeichnet,
daß der flüssigkeitsabsorbierende Kern (4) die flüssigkeitshaltenden Lage (11) und die isolierende Faserlage (12) enthält, die vollständig mit einer flüssigkeitsdurchlässigen Deckschicht (13) bedeckt sind, und die flüssigkeitshaltende Lage (11), die isolierende Faserlage (12) und die flüssigkeitsdurchlässige Deckschicht (13) im wesentlichen über die gesamten Ausdehnungen der jeweiligen Laminierungsoberflächen intermittierend miteinander verbunden sind.

2. Windel nach Anspruch 1, dadurch gekennzeichnet, daß die isolierende Faserlage (12) einen durch ein Schmelzverfahren verbundenen Textilverbundstoff oder Gewebeverbund enthält, und ein Heißkleber oder thermoplastischer Klebstoff (15, 16, 17) als das Bindemittel eingesetzt ist.

3. Windel nach Anspruch 1 oder 2, dadurch gekennzeichnet, daß die isolierende Faserlage (12) eine Dichte hat, die niedriger als jene der bauschigen Pulpe (10) ist, die in der flüssigkeitshaltenden Lage (11) enthalten ist.

4. Windel nach einem der vorhergehenden Ansprüche, dadurch gekennzeichnet, daß die isolierende Faserlage (12) aus einer Mischung aus hydrophilen Fasern und hydrophoben Fasern gebildet ist.

5. Windel nach einem der vorhergehenden Ansprüche, dadurch gekennzeichnet, daß die flüssigkeitsdurchlässige Deckschicht (13) hydrophil ist und ein Körperausscheidungs-Diffusionsvermögen bietet, das höher als jenes ist, das von der isolierenden Faserlage (12) geboten wird.

6. Windel nach einem der vorhergehenden Ansprüche, dadurch gekennzeichnet, daß die flüssigkeitsdurchlässige Deckschicht (13) aus einem Tissue- oder gewebeartigen Papier besteht.

## Claims

1. Disposable diaper, comprising a liquid-permeable topsheet, a liquid-impermeable back sheet and a liquid-absorbent core disposed between said sheets, said core consisting of a liquid-retaining layer comprising fluffy pulp with less than 70% per weight and superabsorbent polymer particles with 30% per weight or more, and an isolating fibre layer which covers the liquid-retaining layer,
characterized in that
the liquid-absorbent core (4) contains said liquid-retaining layer (11) and said isolating fibre layer (12), said layers being covered completely with a liquid-permeable cover sheet (13), and said liquid-retaining layer (11), said isolating fibre layer (12) and said liquid-permeable cover sheet (13) are intermittently bonded together essentially over the entire extension of the respective laminating surfaces.

2. A diaper as claimed in claim 1, characterized in that the isolation fibre layer (12) comprises a nonwoven fabric bonded by melting or a composite fabric, and a hot melt adhesive or a thermoplastic adhesive (15, 16, 17) is used as a bonding means.

3. A diaper as claimed in claim 1 or 2, characterized in that the density of said isolating fibre layer (12) is lower than that of said fluffy pulp (10) contained in said liquid-retaining layer (11).

4. A diaper as claimed in one of the preceding claims, characterized in that said isolating fibre layer (12) is made of a mixture of hydrophilic fibres and hydrophobic fibres.

5. A diaper as claimed in one of the preceding claims, characterized in that said liquid-permeable cover sheet (13) is hydrophilic and provides a diffusibility for bodily excretions higher than that provided by said isolating fibre layer (12).

6. A diaper as claimed in one of the preceding claims, characterized in that said liquid-permeable cover sheet (13) consists of a fabric-like or tissue paper.

## Revendications

1. Une couche a jeter, avec une face intérieure perméable au liquide, une face extérieure imperméable au liquide et un noyau absorbant entre les deux, qui se compose d'une épaisseur retenant le liquide, constituée pour moins de 70% en poids d'une pulpe gonflante et pour 30% en poids ou davantage de particules polymères super-absorbantes, et d'une couche isolante de fibres recouvrant l'épaisseur retenant le liquide,
caractérisé en ce que le noyau absorbant (4) renferme l'épaisseur retenant le liquide (11) et la couche isolante de fibres (12), toutes deux logées sous une feuille de recouvrement perméable au liquide (13), et que l'épaisseur retenant le liquide (11), la couche isolante de fibres (12) et la feuille de recouvrement perméable au liquide (13) sont assemblées de manière intermittente, pour l'essentiel sur toute l'extension des diverses superficies d'enrobage.

2. Une couche a jeter selon la revendication 1, caractérisé en ce que la couche isolante de fibres (12) contient un textile non tissé ou matériau textile aggloméré par un procédé de fusion et que le liant est une colle à chaud ou adhésif thermoplastique (15, 16, 17).

3. Une couche a jeter selon la revendication 1 ou 2, caractérisé en ce que la couche isolante de fibres (12) est plus mince que la couche de pulpe gonflante (10) de l'épaisseur retenant le liquide (11).

4. Une couche a jeter selon l'une quelconque des revendications précédentes, caractérisé en ce que la couche isolante de fibres (12) se compose d'un mélange de fibres hydrophiles et de fibres hydrophobes.

5. Une couche a jeter selon l'une quelconque des revendications précédentes, caractérisé en ce que la feuille de recouvrement perméable au liquide (13) est hydrophile et que sa capacité de diffusion de la déjection corporelle est supérieure à celle de la couche isolante de fibres (12).

6. Une couche a jeter selon l'une quelconque des revendications précédentes, caractérisé en ce que la feuille perméable au liquide (13) se compose d'un tissé en papier.
